# EUROPEAN PATENT APPLICATION

(11) **EP 4 660 203 A1**
(43) Date of publication of application: **10.12.2025**
(21) Application number: 24759423.7
(22) Date of filing: 03.01.2024
(51) Int. Cl.: C07K 16/28, A61K 39/00, A61K 39/395, A61P 35/00

(54) **PHARMACEUTICAL COMBINATION OF ANTI-ALPHA V BETA 3 ANTIBODY AND ANTI-PD-1 ANTIBODY**

(30) Priority: 24.02.2023 CN 202310166943
(71) Applicant: NEWSOARA BIOPHARMA CO., LTD., Shanghai 201413 (CN)
(72) Inventor: WANG, Wenyi, Shanghai 201413 (CN); YIN, Qingqing, Shanghai 201413 (CN); LI, Yuannian, Shanghai 201413 (CN)
(74) Representative: Canzler & Bergmeier Patentanwälte Partnerschaft mbB
(86) International application number: PCT/CN2024/070339
(87) International publication number: WO 2024/174744

(57) **Abstract**

The present disclosure relates to a pharmaceutical combination of an anti-αvβ3 antibody and an anti-PD-1 antibody, a kit, and a method for treating cancer. The pharmaceutical combination comprises an anti-PD-1 antibody and an anti-αvβ3 antibody.

## Description

### TECHNICAL FIELD

The present disclosure relates to the pharmaceutical field, in particular to a pharmaceutical combination of an anti-αvβ3 antibody and an anti-PD-1 antibody, and the use thereof for treating cancers, in particular lung cancers or colon cancers.

### BACKGROUND

Programmed Death-1 (PD-1), a protein receptor identified in 1992, is expressed on the surface of T cells and participates in cellular apoptosis processes. PD-1 possesses two known ligands: PD-L1 (also known as B7-H1 or CD274) and PD-L2 (also known as B7-DC or CD273), which are members of the B7 family expressed on the cell surface (Freeman et al., 2000, J Exp Med 192:1027-34; Latchman et al., 2001, Nat Immunol 2:261-8; Carter et al., 2002, Eur J Immunol 32:634-43). PD-1 immune checkpoint functions as an inhibitory cell surface receptor. Its corresponding ligand, PD-L1, is upregulated on the surface of tumor cells and immune cells within the tumor microenvironment, thereby enabling the tumor cells to evade attack by the immune cells. Blockade of this interaction using anti-PD-1 or anti-PD-L1 antibodies elicits anti-tumor effects.

Therapies based on the PD-1/PD-L1 pathway currently represent a predominant focus within the field of tumor immunotherapy. For instance, immune checkpoint inhibitors directed against this pathway have significantly improved the prognosis of patients with non-small cell lung cancer. However, the majority of patients exhibit poor therapeutic outcomes due to primary resistance. Furthermore, as PD-1/PD-L1 antibodies are used in an increasing number of patients with different types of tumors, researchers have found that anti-PD pathway therapy is not effective for all tumor patients. The PD-1/PD-L1 antibodies are effective only in a subset of patients for each cancer type. For instance, in lung cancer, responses are observed in approximately 30% of patients. This response rate increases to roughly 50% among patients whose tumor tissue tests positive for PD-L1. Conversely, response rates approach 90% in patients with Hodgkin lymphoma, 40-50% in melanoma, and 50% in bladder cancer. Gastrointestinal malignancies, including lung cancer, esophageal carcinoma, and colorectal cancer, exhibit response rates around 30%. Consequently, the discovery of novel therapeutic strategies to enhance the efficacy of anti-PD-1/PD-L1 treatment constitutes an urgent need with high clinical demands and significant innovative potential.

Research has confirmed that integrin αvβ3 plays a crucial role in pathological angiogenesis, such as in lung cancer. The formation of new blood vessels is a hallmark of invasion and metastasis of the tumor. Therefore, targeting integrin αvβ3 to inhibit tumor angiogenesis deprives tumor cells of the necessary blood, oxygen, and other nutrients, thereby promoting apoptosis. Novel monoclonal antibodies directed against αvβ3 have entered the clinical trial stage.

### CONTENT OF THE DISCLOSURE

HY1272 (ABT101) is a novel humanized and affinity-matured IgG4-type antibody based on the previously reported anti-αvβ3 antibody LM609 (see PCT/US2021/028775), and is currently co-developed by our company and a foreign collaborator. The present disclosure is the first to evaluate the tumor treatment effect of a pharmaceutical combination comprising an anti-αvβ3 antibody and an anti-PD-1 antibody. It was discovered that the anti-αvβ3 antibody HY1272 exhibits a significant synergistic effect with an anti-PD-1 antibody. The combination of the both demonstrates a significant tumor growth inhibition against lung cancer cells and colon cancer cells, which are insensitive to monotherapy of the anti-PD-1 antibody. This suggests that HY1272 has a synergistic effect with the anti-PD-1 antibodies and is expected to further improve tumor treatment effect beyond the anti-PD-1 monotherapy.

The present disclosure satisfies the aforementioned need by providing a pharmaceutical combination comprising an anti-PD-1 antibody and an anti-αvβ3 antibody. In one aspect, the present disclosure provides a pharmaceutical combination comprising an anti-αvβ3 antibody and an anti-PD-1 antibody.

In some embodiments, the anti-αvβ3 antibody comprises:
a HCDR1 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 1; a HCDR2 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 2; a HCDR3 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 3; and
a LCDR1 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 4; a LCDR2 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 5; a LCDR3 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 6.

In some embodiments, the anti-αvβ3 antibody comprises:
a HCDR1 region comprising the amino acid sequence set forth in SEQ ID NO: 1; a HCDR2 region comprising the amino acid sequence set forth in SEQ ID NO: 2; a HCDR3 region comprising the amino acid sequence set forth in SEQ ID NO: 3; and
a LCDR1 region comprising the amino acid sequence set forth in SEQ ID NO: 4; a LCDR2 region comprising the amino acid sequence set forth in SEQ ID NO: 5; a LCDR3 region comprising the amino acid sequence set forth in SEQ ID NO: 6.

In some specific embodiments, the anti-αvβ3 antibody comprises a heavy chain variable region comprising the amino acid sequence set forth in SEQ ID NO: 7 and a light chain variable region comprising the amino acid sequence set forth in SEQ ID NO: 8.

In some specific embodiments, the anti-αvβ3 antibody is HY1272.

In some specific embodiments, the anti-PD-1 antibody is, for example, purchased from Bioxcell (Catalog No. BE0146); or Keytruda^{®} (Pembrolizumab), Opdivo^{®} (Nivolumab), or the like.

In some embodiments, the anti-PD-1 antibody and the anti-αvβ3 antibody are each in a pharmaceutically acceptable form and can be administered simultaneously, sequentially, or separately.

In some specific embodiments, the anti-PD-1 antibody and the anti-αvβ3 antibody are each in a pharmaceutically acceptable form and are administered simultaneously.

In some embodiments, the route of administration for the pharmaceutical combination comprises intravenous injection, intraperitoneal injection, intramuscular injection, subcutaneous injection, oral administration, sublingual administration, nasal administration, or transdermal administration.

In some specific embodiments, the route of administration for the pharmaceutical combination is intravenous injection.

In one aspect, the present disclosure provides a use of the pharmaceutical combination described above in the manufacture of a medicament for treating a patient suffering from a cancer.

In some embodiments, the cancer is a lung cancer or a colon cancer.

In some specific embodiments, the tumor tissue section of the patient is positive for PD-L1 expression, optionally positive for αvβ3 expression.

In one aspect, the present disclosure provides a method of treating cancer, comprising administering a therapeutically effective amount of the pharmaceutical combination described above to a patient suffering from a cancer.

In some embodiments, the cancer is a lung cancer or a colon cancer.

In some specific embodiments, the tumor tissue section of the patient is positive for PD-L1 expression, optionally positive for αvβ3 expression.

In one aspect, the present disclosure provides a use of an anti-αvβ3 antibody in the manufacture of a pharmaceutical combination for combined use with an anti-PD-1 antibody.

In some embodiments, the anti-αvβ3 antibody comprises:
a HCDR1 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 1; a HCDR2 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 2; a HCDR3 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 3; and
a LCDR1 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 4; a LCDR2 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 5; a LCDR3 region having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 6.

In some embodiments, the anti-αvβ3 antibody comprises:
a HCDR1 region comprising the amino acid sequence set forth in SEQ ID NO: 1; a HCDR2 region comprising the amino acid sequence set forth in SEQ ID NO: 2; a HCDR3 region comprising the amino acid sequence set forth in SEQ ID NO: 3; and
a LCDR1 region comprising the amino acid sequence set forth in SEQ ID NO: 4; a LCDR2 region comprising the amino acid sequence set forth in SEQ ID NO: 5; a LCDR3 region comprising the amino acid sequence set forth in SEQ ID NO: 6.

In some specific embodiments, the anti-αvβ3 antibody comprises a heavy chain variable region comprising the amino acid sequence set forth in SEQ ID NO: 7 and a light chain variable region comprising the amino acid sequence set forth in SEQ ID NO: 8.

In some specific embodiments, the anti-αvβ3 antibody is HY1272.

In one aspect, the present disclosure provides a combination therapy for treating a patient suffering from a cancer, comprising administering a therapeutically effective amount of the anti-αvβ3 antibody described above alone to said patient, as well as administering a therapeutically effective amount of an anti-PD-1 antibody alone.

In some embodiments, the cancer is a lung cancer or a colon cancer.

In some specific embodiments, the tumor tissue section of the patient is positive for PD-L1 expression, optionally positive for αvβ3 expression.

In some specific embodiments, the anti-αvβ3 antibody is administered within ±5 minutes of administering the anti-PD-1 antibody.

The superior technical effects of the pharmaceutical combination, the kit, and the treating method described in the present disclosure mainly lie in the following aspects:
(1) Compared to administering any single drug in the combination alone, it produces a better therapeutic effect in terms of reducing tumor growth or even eliminating tumors.
(2) Compared to administering any single drug in the combination alone, it allows for administration of a reduced amount.
(3) It provides a better disease control rate among treated patients.
(4) It provides a longer survival (e.g., median survival, progression-free survival, or overall survival) among the treated patients.
(5) Compared to standard chemotherapy, it provides a longer survival (e.g., median survival, progression-free survival, or overall survival) among the treated patients.
(6) Compared to administering any single drug in the combination alone, it exhibits a good activity in treating tumors or proliferative diseases, demonstrating superior synergistic anti-tumor effects.

### BRIEF DESCRIPTION OF THE DRAWINGS

Figure 1: Animal tumor volume at each time point in each group of the LLC-hITGB3 subcutaneous xenograft model. *: P < 0.05, : P < 0.01, compared to the vehicle group.
Figure 2: Animal tumor volume at each time point in each group of the CT26-hITGB3 subcutaneous xenograft model. *: P < 0.05, : P < 0.01, compared to the vehicle group.

### DETAILED DESCRIPTION OF THE PREFERRED EMBODIMENT

Although the present disclosure may be implemented in many different forms, the specific illustrative embodiments disclosed herein are to verify the principles of thisdisclosure. It should be emphasized that the present disclosure is not limited to the specific embodiments illustrated. In addition, any section headings used herein are for organizational purposes only and are not to be interpreted as limiting the subject matter described.

Unless otherwise defined herein, scientific and technical terms used in connection with the present disclosure shall have the meanings commonly understood by one skilled in the art. Moreover, unless the context requires otherwise, singular terms shall include plural forms and plural terms shall include the singular form. More specifically, as used in this specification and the appended claims, the singular forms "a", "an", and "the" include plural referents unless the context clearly indicates otherwise. In this application, unless otherwise stated, the use of "or" means "and/or". Furthermore, the use of the term "comprising" and other forms (such as "including" and "containing") is not limiting. Additionally, ranges provided in the specification and the appended claims include the endpoints and all values between the endpoints.

Generally, terminology related to the cell and tissue culture, molecular biology, immunology, microbiology, genetics, and protein and nucleic acid chemistry and hybridization described herein, and the techniques involved, are well known and commonly used in the art. Unless otherwise indicated, the methods and techniques of the present disclosure are generally performed according to conventional methods well known in the art and as described in the various general and more specific references that are cited and discussed throughout the present specification. See, e.g., Sambrook J. & Russell D., Molecular Cloning: A Laboratory Manual, 3rd Ed., Cold Spring Harbor Laboratory Press, Cold Spring Harbor, N.Y. (2000); Abbas et al., Cellular and Molecular Immunology, 6th Ed., W.B. Saunders Company (2010); Harlow and Lane, Using Antibodies: A Laboratory Manual, Cold Spring Harbor Laboratory Press, Cold Spring Harbor, N.Y. (1998); Ausubel et al., Short Protocols in Molecular Biology: A Compendium of Methods from Current Protocols in Molecular Biology, Wiley, John & Sons, Inc. (2002); and Coligan et al., Short Protocols in Protein Science, Wiley, John & Sons, Inc. (2003). Terminology related to analytical chemistry, synthetic organic chemistry, and pharmaceutical and medicinal chemistry described herein, and laboratory procedures and techniques, are well known and commonly used in the art. Furthermore, any section headings used herein are for organizational purposes only and are not to be interpreted as limiting the described subject matter.

### Definitions

For a better understanding of the present disclosure, definitions and explanations of related terms are provided below. Unless otherwise specified, the following terms used in the present disclosure have the meanings given below. A particular term not specifically defined should not be considered indefinite or unclear, but should be understood according to its ordinary meaning in the art. When a trade name appears in the present disclosure, it is intended to refer to the corresponding commercial product, composition, or active ingredient thereof.

As used herein, the term "antibody" refers to an antigen-binding protein having at least one antigen-binding domain. The antibodies and fragments thereof of the present disclosure may be whole antibodies or any fragments thereof. Thus, the antibodies and fragments thereof of the present disclosure include monoclonal antibodies or fragments thereof and antibody variants or fragments thereof, as well as immunoconjugates. Examples of antibody fragments include Fab fragments, Fab' fragments, F(ab)'2 fragments, Fv fragments, isolated CDR regions, single-chain Fv molecules (scFv), and other antibody fragments known in the art. Antibodies and fragments thereof may also include recombinant polypeptides, fusion proteins, and bispecific antibodies. The anti-PD-1 antibody and anti-αvβ3 antibody disclosed herein may be IgG1, IgG2, IgG3, or IgG4 isotypes. The term "isotype" refers to the type of antibody encoded by the heavy chain constant region gene. Generally see, Fundamental Immunology Ch. 7 (Paul, W., ed., 2nd ed. Raven Press, N.Y. (1989)).

As used herein, the term "CDR" or "CDRs" means the complementarity determining region(s) in an immunoglobulin variable region as defined using the Kabat numbering system, unless otherwise indicated.

As used herein, the term "treat," "treating" and "treatment" generally refers to an operation to obtain the desired pharmacological and/or physiological effect. The effect may be prophylactic in terms of completely or partially preventing a disease or its symptoms; and/or it may be therapeutic in terms of partially or completely stabilizing or curing the disease and/or side effects arising from the disease. "Treat," "treating" and "treatment" as used herein encompass any treatment of a patient's disease, including: (a) inhibiting the symptoms of the disease, i.e., preventing its development; or (b) alleviating the symptoms of the disease, i.e., causing regression of the disease or the symptoms.

As used herein, the term "administer," "administering" and "administration" means physically introducing a composition comprising a therapeutic agent to a subject using any of various methods and delivery systems known to those skilled in the art. Routes of administration include intravenous, intramuscular, subcutaneous, intraperitoneal, spinal, or other parenteral routes of administration, for example, by injection or infusion. The phrase "parenteral administration" as used herein means a mode of administration other than enteral administration, usually by injection, and topical administration, and includes, but is not limited to, intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion, as well as *in vivo* electroporation. In certain embodiments, administration is via a non-parenteral route. In certain embodiments, it is administered orally. Other non-parenteral routes include topical, epidermal, or mucosal routes of administration, e.g., intranasally, vaginally, rectally, sublingually, or topically. Administration may also be performed, for example, once, multiple times, and/or over one or more extended periods.

As used herein, "co-administration" of agents such as a PD-1 antagonist or αVβ3 antagonist means administering the agents such that they have overlapping therapeutic activity, and does not necessarily mean that the agents are administered simultaneously to the subject. The agents may or may not be physically combined prior to administration. In one embodiment, the agents are administered to the subject simultaneously or approximately simultaneously. For example, anti-PD-1 antibody and anti-αVβ3 antibody drug products contained in separate vials, when in a liquid solution, can be mixed into the same intravenous infusion bag or injection device, and administered simultaneously to the patient.

As used herein, the term "immunotherapy" means treating a subject having a disease or at risk of infection or suffering a relapse of a disease by a method that includes inducing, enhancing, suppressing, or otherwise modifying an immune response. "Treatment" or "therapy" of a subject means any type of intervention or procedure performed on the subject, or administration of an active agent to the subject, with the purpose of reversing, alleviating, ameliorating, inhibiting, slowing, or preventing the onset, progression, development, severity, or relapse of symptoms, complications, or conditions, or biochemical markers associated with the disease.

As used herein, the terms "identity" and "similarity" refer to the relationship between the sequences of two or more polypeptide molecules or two or more nucleic acid molecules as determined by aligning and comparing the sequences. "Percentage identity" refers to the percentage of identical residues between amino acids or nucleotides in the molecules being compared and is calculated based on the size of the smallest molecule being compared. For these calculations, gaps in the alignment (if any) are preferably addressed by specific mathematical models or computer programs (i.e., "algorithms"). Methods that can be used to calculate the identity of nucleic acids or polypeptides include those described in Computational Molecular Biology, (Lesk, A.M. ed.), 1988, New York: Oxford University Press; Biocomputing: Informatics and Genome Projects, (Smith, D.W. ed.), 1993, New York: Academic Press; Computer Analysis of Sequence Data, Part I, (Griffin, A.M. and Griffin, H.G. eds.), 1994, New Jersey: Humana Press; von Heinje, G., 1987, Sequence Analysis in Molecular Biology, New York: Academic Press; Sequence Analysis Primer, (Gribskov, M. and Devereux, J. eds.), 1991, New York: M. Stockton Press; and Carillo et al., 1988, SIAM J. Applied Math. 48:1073.

As used herein, the term "individual" includes any human or non-human animal. The term "non-human animal" includes, but is not limited to, vertebrates such as non-human primates, sheep, dogs, and rodents such as mice, rats, and guinea pigs. In certain embodiments, the subject is a human. The "subject" means a mammal, such as a rodent, feline, canine, and primate. Preferably, the subject according to the present disclosure is a human. The terms "individual", "subject", and "patient" may be used interchangeably herein in certain contexts.

As used herein, a "therapeutically effective amount" or "therapeutically effective dose" of a drug or a therapeutic agent is any amount of the drug that, when used alone or in combination with another therapeutic agent, protects a subject from the onset of a disease or promotes disease regression, as evidenced by a decrease in the severity of disease symptoms, an increase in the frequency and duration of disease symptom-free phases, or the prevention of injury or disability caused by the affliction of the disease. The amount of an active compound or a material, composition, or dosage form containing an active compound is effective to produce certain desired therapeutic effects commensurated with a reasonable benefit/risk ratio when administered in accordance with the desired therapeutic regimen.

As an example for treating cancer, a therapeutically effective amount of an anticancer agent may inhibit cell growth or tumor growth by at least about 10%, at least about 20%, at least about 40%, at least about 60%, or at least about 80% relative to an untreated subject or, in certain embodiments, relative to patients treated with a standard care therapy. In other embodiments of the present disclosure, tumor regression may be observed and sustained for a period of at least about 20 days, at least about 40 days, or at least about 60 days. Despite these ultimate measures of therapeutic effectiveness, the evaluation of immunotherapeutic drugs must also consider "immune-related" response patterns.

As used herein, the term "cancer" refers to a large group of various diseases characterized by uncontrolled growth of abnormal cells in the body. It refers to any tumor or malignant cell growth, proliferation, or metastasis-mediated solid and non-solid tumors causing a medical disorder. A "recurrent" cancer is a cancer that regenerates at the original site or a distant site after a response to an initial treatment (e.g., surgery). A "locally recurrent" cancer is a cancer that occurs at the same site as a previously treated cancer after treatment. A "metastatic" cancer refers to a cancer that spreads from one part of the body (e.g., the lung) to another part of the body.

As used herein, the term "PD-L1 expression" means any detectable level of expression of a designated PD-L protein on the cell surface or of a designated PD-L mRNA within a cell or tissue. PD-L protein expression can be detected in an IHC assay on tumor tissue sections or by flow cytometry using diagnostic PD-L antibodies. Alternatively, PD-L protein expression on tumor cells can be detected by PET imaging, using binding agents (e.g., antibody fragments, affibodies, etc.) that specifically bind to the desired PD-L target (e.g., PD-L1). Techniques for detecting and measuring PD-L mRNA expression include RT-PCR, real-time quantitative RT-PCR, RNAseq, and the Nanostring platform (J. Clin. Invest. 2017;127(8):2930-2940).

Several methods have been described for quantifying PD-L1 protein expression in IHC assays on tumor tissue sections. See, e.g., Thompson, R. H., et al., PNAS 101 (49); 17174-17179 (2004); Thompson, R. H., et al., Cancer Res. 66:3381-3385 (2006); Gadiot, J., et al., Cancer 117:2192-2201 (2011); Taube, J. M. et al., Sci Transl Med 4, 127ra37 (2012); and Toplian, S. L. et al., New Eng. J Med. 366 (26): 2443-2454 (2012). See US20170285037, which describes hematoxylin and eosin staining used by pathologists.

One method employs a simple binary endpoint of positive or negative for PD-L1 expression, where a positive result is defined in terms of the percentage of tumor cells exhibiting histological evidence of cell-surface membrane staining. A tumor tissue section is counted as positive for PD-L1 expression if PD-L1 expression is at least 1% of the total tumor cells.

In another method, PD-L1 expression in a tumor tissue section is quantified both in the tumor cells and in infiltrating immune cells primarily comprising lymphocytes. The percentage of tumor cells and infiltrating immune cells exhibiting membrane staining is quantified as <5%, 5% to 9%, and then in 10% increments up to 100%. PD-L1 expression in the immune infiltrate is reported as a semi-quantitative measurement named the adjusted inflammation score (AIS), which is determined by multiplying the percentage of membrane-stained cells by the intensity of the infiltrate, which is graded as none (0), mild (score of 1, rare lymphocytes), moderate (score of 2, focal infiltration of tumor by lymphohistiocytic aggregates), or severe (score of 3, diffuse infiltration). A tumor tissue section is counted as positive for PD-L1 expression by the immune infiltrate if the AIS is ≥5.

The level of PD-L mRNA expression can be compared to the mRNA expression levels of one or more reference genes that are frequently used in quantitative RT-PCR.

As used herein, the term "positive for PD-L1 expression" refers to an elevated level of PD-L1 expression (protein and/or mRNA) in malignant cells and/or infiltrating immune cells within the tumor, compared to an appropriate control.

As used herein, the term "αvβ3 expression" means any detectable level of expression of a designated αvβ3 protein on the cell surface or of a designated αvβ3 mRNA within a cell or tissue. αvβ3 protein expression can be detected in an IHC assay on tumor tissue sections or by flow cytometry using a diagnostic anti-αvβ3 antibody. Alternatively, αvβ3 protein expression on tumor cells can be detected by PET imaging, using binding agents (e.g., antibody fragments, affibodies, etc.) that specifically bind to αvβ3. Techniques for detecting and measuring αvβ3 mRNA expression include RT-PCR, real-time quantitative RT-PCR, RNAseq, and the Nanostring platform (J. Clin. Invest. 2017;127(8):2930-2940).

As used herein, the term "positive for αvβ3 expression" means the percentage of αvβ3 positive cells or the percentage of CPS-like αvβ3 positive cells ≥1%.

As used herein, any concentration range, percentage range, ratio range, or integer range shall be understood to include the value of any integer within the recited range and, where appropriate, fractions thereof (such as one tenth and one hundredth of an integer), unless otherwise indicated.

As used herein, the term "pharmaceutically acceptable" means that a vehicle, diluent, excipient, and/or salt thereof is chemically and/or physically compatible with the other ingredients in the formulation and is physiologically compatible with the recipient.

As used herein, the term "pharmaceutically acceptable carrier and/or excipient" means a carrier and/or excipient that is pharmacologically and/or physiologically compatible with the subject and the active agent, which are well known in the art (see, e.g., Remington's Pharmaceutical Sciences. Edited by Gennaro AR, 19th Ed., Pennsylvania: Mack Publishing Company, 1995), and include, but are not limited to, pH adjusters, surfactants, adjuvants, and ionic strength enhancers. For example, the pH adjusters include, but are not limited to, phosphate buffers; the surfactants include, but are not limited to, cationic, anionic, or non-ionic surfactants, such as Tween-80; and the ionic strength enhancers include, but are not limited to, sodium chloride.

As used herein, the terms "combined use", "used in combination", or "combination therapy" mean that two or more active substances can be administered to a subject together in a mixture, simultaneously each as a single formulation, or sequentially each as a single formulation in any order.

As used herein, the term "pharmaceutical combination" refers to combination therapy, combined therapy, or combined medication treatment, which uses more than one drug to treat a tumor, i.e., a single disease. In the present disclosure, "pharmaceutical combination" is used for combination therapy, which comprises administering an αVβ3 antagonist (e.g., anti-αVβ3 antibody) and a PD-1 antagonist (e.g., anti-PD-1 antibody) for the treatment of cancer (such as a lung cancer or a colon cancer). In contrast, "monotherapy" refers to a treatment method using a single drug alone (e.g., anti-αVβ3 antibody or anti-PD-1 antibody).

### Route of Administration

The following content does not limit the route of administration of the pharmaceutical combination of the present disclosure.

The components of the pharmaceutical combination of the present disclosure can each be formulated separately, or some or all of them can be co-formulated. In one embodiment, the pharmaceutical combination of the present disclosure can be formulated as a pharmaceutical combination suitable for single or multiple administrations.

The components of the pharmaceutical combination of the present disclosure can each be administered independently, or some or all of them can be co-administered. The components of the pharmaceutical combination of the present disclosure may not be administered substantially simultaneously, or some or all of them may be administered substantially simultaneously.

The components of the pharmaceutical combination of the present disclosure can each be administered independently, or some or all of them can be co-administered, via various suitable routes, including but not limited to, oral or parenteral (via intravenous, intramuscular, topical, or subcutaneous routes). In some embodiments, the components of the pharmaceutical combination of the present disclosure can each be administered independently, or some or all of them can be co-administered orally or by injection, e.g., intravenous injection or intraperitoneal injection.

The components of the pharmaceutical combination of the present disclosure can each be independently, or some or all of them together, in suitable dosage forms, including but not limited to, tablets, lozenges, pills, capsules (e.g., hard capsules, soft capsules, enteric-coated capsules, microcapsules), elixirs, granules, syrups, injections (intramuscular, intravenous, intraperitoneal), granules, emulsions, suspensions, solutions, dispersions, and dosage forms for sustained-release formulations for oral or non-oral administration.

The components of the pharmaceutical combination of the present disclosure can each independently, or some or all of them together, contain a pharmaceutically acceptable carrier and/or excipient.

### Sequence Listing Summary

The present application is accompanied by a Sequence Listing containing numerous nucleic acid and amino acid sequences. Table 1 below provides a summary of the included sequences.

**Table 1**

| SEQ ID NO: | Sequence | Name |
|---|---|---|
| 1 | SYDMS | HY1272 HCDR1 sequence |
| 2 | KVSSGGGSTYYLDTVQG | HY1272 HCDR2 sequence |
| 3 | HLHGSFAS | HY1272 HCDR3 sequence |
| 4 | QASQSISNFLH | HY1272 LCDR1 sequence |
| 5 | YRSQSIS | HY1272 LCDR2 sequence |
| 6 | QQSGSWPLT | HY1272 LCDR3 sequence |
| 7 | QVQLVESGGGVVQPGRSLRLSCAASGFTFSS YDMSWVRQAPGKGLEWVAKVSSGGGSTYYL DTVQGRFTISRDNSKNTLYLQMNSLRAEDTAV YYCARHLHGSFASWGQGTTVTVSS | HY1272 Heavy chain variable region sequence |
| 8 | EIVLTQSPATLSLSPGERATLSCQASQSISNFLH WYQQRPGQAPRLLIRYRSQSISGIPARFSGSGS GTDFTLTISSLEPEDFAVYYCQQSGSWPLTFGG GTKVEIK | HY1272 Light chain variable region sequence |
| 9 | ESKYGPPCPPCPAPEFLGGPSVFLFPPKPKD TLMISRTPEVTCVVVDVSQEDPEVQFNWYVD GVEVHNAKTKPREEQFNSTYRVVSVLTVLHQ DWLNGKEYKCKVSNKGLPSSIEKTISKAKGQP REPQVYTLPPSQEEMTKNQVSLTCLVKGFYP SDIAVEWESNGQPENNYKTTPPVLDSDGSFF LYSRLTVDKSRWQEGNVFSCSVMHEALHNH YTQKSLSLSLGK | Human IgG4 Fc region (S228P) sequence |
| 10 | QVQLVESGGGVVQPGRSLRLSCAASGFTFSSY DMSWVRQAPGKGLEWVAKVSSGGGSTYYLDT VQGRFTISRDNSKNTLYLQMNSLRAEDTAVYYC ARHLHGSFASWGQGTTVTVSSASTKGPSVFPL APCSRSTSESTAALGCLVKDYFPEPVTVSWNS GALTSGVHTFPAVLQSSGLYSLSSVVTVPSSSL GTKTYTCNVDHKPSNTKVDKRVESKYGPPCPP CPAPEFLGGPSVFLFPPKPKDTLMISRTPEVTC VVVDVSQEDPEVQFNWYVDGVEVHNAKTKPR EEQFNSTYRVVSVLTVLHQDWLNGKEYKCKVS NKGLPSSIEKTISKAKGQPREPQVYTLPPSQEE MTKNQVSLTCLVKGFYPSDIAVEWESNGQPEN NYKTTPPVLDSDGSFFLYSRLTVDKSRWQEGN VFSCSVMHEALHNHYTQKSLSLSLGK | HY1272 Heavy chain sequence |
| 11 | EIVLTQSPATLSLSPGERATLSCQASQSISNFLH WYQQRPGQAPRLLIRYRSQSISGIPARFSGSGS GTDFTLTISSLEPEDFAVYYCQQSGSWPLTFGG GTKVEIKRTVAAPSVFIFPPSDEQLKSGTASVVC LLNNFYPREAKVQWKVDNALQSGNSQESVTEQ DSKDSTYSLSSTLTLSKADYEKHKVYACEVTHQ GLSSPVTKSFNRGEC | HY1272 Light chain sequence |
| 12 | CAAGTGCAGCTGGTGGAATCCGGCGGAGG AGTCGTGCAGCCTGGCAGATCCCTGCGGCT GTCCTGTGCTGCTAGTGGATTTACCTTCAG CTCCTACGACATGTCCTGGGTGCGGCAGGC TCCAGGAAAAGGCCTGGAATGGGTGGCCAA GGTGTCCTCTGGCGGCGGCTCTACCTACTA CCTGGACACCGTCCAAGGCCGCTTCACCAT CTCTCGGGACAACTCTAAGAACACCCTGTA TCTGCAGATGAACTCGCTGAGAGCCGAGGA CACCGCTGTGTACTACTGCGCCAGACACCT GCACGGCAGCTTCGCCTCTTGGGGACAGG GCACCACCGTGACCGTGTCCAGCGCCAGC ACCAAGGGCCCTTCCGTGTTCCCTCTCGCC CCATGCTCCCGGTCTACCTCCGAGTCTACC GCTGCCCTGGGCTGCCTGGTGAAGGACTA CTTCCCCGAACCTGTGACAGTCAGCTGGAA CTCCGGCGCTCTGACCTCTGGCGTGCACAC CTTTCCTGCCGTGCTGCAGTCCAGCGGCCT GTACTCTCTGTCTTCCGTGGTGACCGTGCC TTCCAGCTCTCTCGGCACCAAGACCTACAC CTGCAACGTGGACCACAAGCCTAGCAATAC | HY1272 Heavy chain nucleotide sequence |
| | CAAAGTGGATAAAAGAGTGGAGTCCAAGTA CGGCCCACCTTGCCCTCCTTGTCCTGCTCC TGAGTTCCTGGGCGGACCTTCTGTGTTTCT GTTCCCTCCTAAGCCCAAGGATACCCTGAT GATCTCCCGGACCCCTGAGGTGACTTGCGT GGTGGTTGACGTGTCTCAGGAAGATCCTGA AGTGCAGTTCAACTGGTACGTGGATGGCGT GGAAGTGCACAACGCCAAGACAAAGCCTAG AGAGGAACAGTTCAACTCCACCTACAGAGT GGTCTCCGTGCTGACCGTGCTGCATCAGGA CTGGCTGAACGGCAAAGAGTACAAGTGCAA GGTGTCCAACAAGGGCCTGCCCTCTTCCAT CGAGAAGACAATCTCCAAGGCCAAAGGCCA GCCTCGGGAGCCTCAGGTTTATACACTGCC TCCATCTCAAGAAGAGATGACCAAGAACCA GGTGTCTTTGACCTGTCTGGTCAAGGGCTT CTACCCCTCTGACATCGCCGTCGAGTGGGA GTCTAATGGCCAGCCCGAGAACAACTACAA GACAACACCCCCCGTGCTGGATTCCGACGG CTCCTTCTTCCTGTACTCCAGACTGACAGTG GACAAGTCCAGATGGCAGGAGGGCAACGT GTTCTCCTGCTCTGTGATGCACGAGGCTTT GCATAACCACTACACCCAGAAGTCCCTGAG CCTGAGCCTGGGTAAG | |
| 13 | GAGATCGTGCTGACACAGTCTCCTGCTACC CTGTCCCTGAGCCCCGGAGAACGGGCTAC ACTGAGCTGTCAGGCCTCTCAGAGCATCTC CAATTTTCTGCACTGGTACCAGCAGCGGCC AGGCCAGGCTCCTCGCCTGTTGATCAGATA TAGATCCCAGTCCATCTCCGGCATCCCCGC TAGATTCTCCGGCTCTGGATCTGGCACCGA TTTCACCCTGACCATCAGCAGCCTGGAACC TGAGGACTTCGCCGTGTACTACTGCCAACA GAGTGGCTCTTGGCCTCTGACCTTCGGCGG CGGAACTAAAGTGGAAATCAAGCGGACCGT GGCCGCTCCTTCTGTGTTCATCTTTCCTCCT AGCGATGAACAGCTGAAGTCTGGCACCGCC TCTGTGGTCTGCCTGCTGAACAACTTCTACC CCAGAGAGGCCAAAGTGCAGTGGAAGGTG GACAACGCCCTGCAGTCCGGCAACTCCCAA GAGTCCGTGACCGAGCAGGACTCCAAGGA CTCTACCTACTCCCTGTCCTCCACCCTGACA CTGTCTAAGGCCGACTACGAGAAGCACAAG GTGTACGCCTGCGAGGTGACCCATCAGGG CCTGTCCTCTCCTGTGACCAAGTCCTTCAAC AGAGGCGAGTGC | HY1272 light chain nucleotide sequence |

The following describes the present disclosure in conjunction with specific examples for further illustration. However, these examples in the present disclosure are used for clarification only and do not limit the scope of the present disclosure. Similarly, the present disclosure is not limited to any specific preferred embodiments described herein. Those skilled in the art should understand that equivalent substitutions or corresponding modifications made to the technical features of the present disclosure still fall within the scope of protection of the present disclosure. Unless otherwise specified, the reagents used in the following examples are commercially available products, and the preparation of solutions can be performed using conventional techniques in the art.

### Example 1: Efficacy Study of HY1272 in Combination with Anti-PD1 Antibody in Mouse Lung Cancer LLC-hITGB3 Cell Line of C57BL/6 Mouse Subcutaneous Xenograft Model

### 1.1 Construction of LLC-ITGB3 Stable Cell Line

This experiment utilized a hITGB3 (human integrin β3) plasmid packaged into a lentivirus to infect LLC mouse Lewis lung cancer cells, thereby constructing a stable cell line expressing hITGB3 (LLC-hITGB3). The cell line was validated using flow cytometry, with a positive rate exceeding 95%, and the LLC-hITGB3 cell line was successfully constructed. The method was as follows:
The human ITGB3 gene was cloned into the pLVX-PGK-Puro vector using gene synthesis method. Lentivirus was produced using a three-plasmid system. Stable overexpression cell lines were generated via lentiviral infection. Briefly, LLC cells in logarithmic growth phase were seeded at a density of 1×10⁵ cells/mL, with 1 mL per well in a 12-well plate. The viral solution was added to the cell culture plate and incubated in a 37°C incubator with 5% CO₂ for 24 hours. After incubation, the supernatant was discarded, a complete medium (DMEM + 10% FBS) was added, and the plate was incubated for another 48 hours. After the incubation, the supernatant was discarded, and the cell pool was subjected to pressure selection using 5 µg/mL puromycin for at least 3 days. Subsequently, the cells were gradient diluted and seeded at a dilution of 1 cell per well in a 96-well cell culture plate. Single clones were expanded and subjected to flow cytometry detection. If the positive rate of a clone was below 90% after detection, the puromycin concentration was increased for further selection to obtain a clone with a positive rate greater than 90%. Finally, after further screening using two higher concentrations of puromycin (16 µg/mL and 32 µg/mL), a cell pool with a high positive rate of up to 98.85% was obtained, successfully constructing the stable cell line LLC-hITGB3.

The culture supernatant of the constructed stable cell line LLC-ITGB3 was tested for mycoplasma using a mycoplasma detection kit (Lonza MycoAlert^{™} mycoplasma detection kit, Cat. No. LT07-318) according to the manufacturer's instructions. The test results showed that the cells were negative for mycoplasma.

### 1.2 Study of the Anti-Tumor Efficacy of Test Substances in Mouse Lung Cancer LLC-hITGB3 Cell Line of C57BL/6 Mouse Subcutaneous Xenograft Model

The mouse lung cancer LLC-hITGB3 cell line was cultured in a DMEM medium supplemented with 10% FBS in a 37°C incubator with 5% CO₂. Cells were collected before continuous passage beyond ten generations. Cells (5 × 10⁵/100 µL) were subcutaneously injected into the right back near the armpit of female C57BL/6 mice. Mice were anesthetized with 3-4% isoflurane prior to inoculation. When the tumor volume (TV) reached an average of approximately 50 mm³, mice were randomly divided into 4 groups (n=6 per group) based on tumor volume and body weight. The day of grouping and dosing was defined as Day 0. Grouping and dosing regimens are shown in Table 2.

**Table 2. Grouping and Dosing Regimen**

| **Group** | **Test Substance** | **Number of Animal** | **Dose (mg/kg)** | **Dosing Regimen** |
|---|---|---|---|---|
| 1 | Vehicle | 6 | - | i.p. injection, twice a week |
| 2 | Anti-Mouse PD-1 | 6 | 10 | i.p. injection, twice a week |
| 3 | HY1272 | 6 | 10 | i.p. injection, twice a week |
| 4 | Anti-Mouse PD-1+HY1272 | 6 | 10+10 | i.p. injection, twice a week |

| | | | | |
|---|---|---|---|---|
| *The anti-mouse PD-1 antibody was purchased from Bioxcell (Catalog No. BE0146). | | | | |

The body weight and tumor volume of the mouse were measured and recorded twice a week after grouping. Tumor volume (TV) was calculated as follows: TV = (length × width²) / 2. Based on the measured tumor volumes, the relative tumor growth inhibition (TGI) was calculated using the following formula: TGI % = (1 - T/C) × 100%, where T and C are the tumor volumes (TV) of the experimental group and the vehicle control group, respectively, at a specific time point.

The mean TV value for each group is presented as mean ± standard error of the mean (SEM). Comparisons between groups were performed using Dunnett's multi-comparison test. P-value < 0.05 was considered statistically significant.

### 1.3 Experimental Results

Under these experimental conditions, LLC-hITGB3 cells exhibited normal growth, and tumor growth proceeded normally. Under these experimental conditions, there was no significant difference in body weight among the experimental groups compared to the vehicle control group at the same time point during the dosing period.

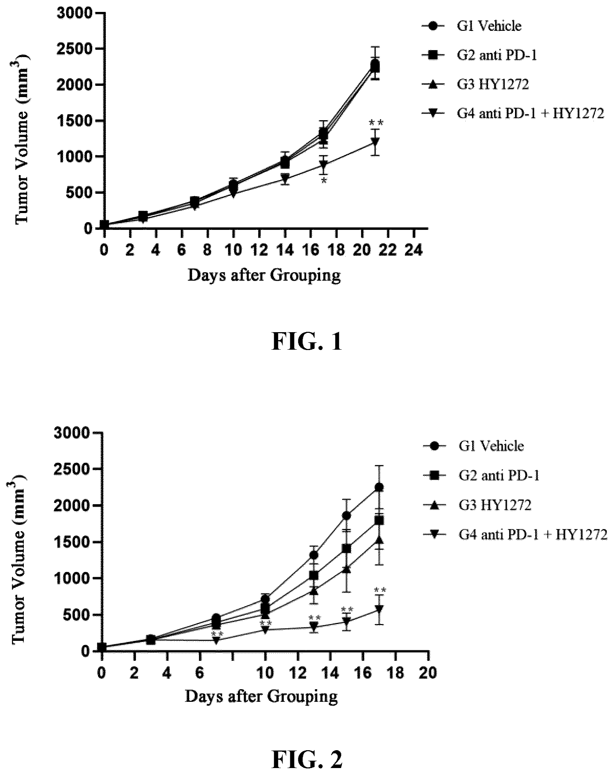

The tumor growth curve in mice is shown in Figure 1. Compared to the vehicle control group at the same time point, on Day 21, the tumor growth inhibition (TGI) for the anti-PD-1 monotherapy group (10 mg/kg) and the HY1272 monotherapy group (10 mg/kg) was 2.8% and 2.5%, respectively, showing no significant difference compared to the vehicle control group. The TGI for the combination therapy group (10 mg/kg anti-PD-1 + 10 mg/kg HY1272) was 47.9%, showing a significant difference compared to the vehicle control group, the anti-PD-1 monotherapy group, or the HY1272 monotherapy group.

These results indicate that in this lung cancer model, neither anti-PD-1 monotherapy nor HY1272 monotherapy showed significant anti-tumor effects, but their combination exhibited a significant anti-tumor effect. This suggests that HY1272 has a synergistic effect with anti-PD-1 and can significantly enhance the sensitivity of certain lung cancer cells to anti-PD-1 therapy.

### Example 2: Efficacy Study of HY1272 in Combination with Anti-PD1 Antibody in Mouse Colon Cancer CT26-hITGB3 Cell Line of BALB/c Mouse Subcutaneous Xenograft Model

### 2.1 Construction of CT26-ITGB3 Stable Cell Line

This experiment utilized an ITGB3 plasmid packaged into a lentivirus to infect CT26 mouse colon cancer cells, thereby constructing a stable cell line expressing hITGB3 (CT26-hITGB3). The cell line was validated using flow cytometry, with a positive rate exceeding 95%, and the CT26-hITGB3 cell line was successfully constructed. The method was as follows:
The human ITGB3 gene was cloned into the pLVX-PGK-Puro vector using gene synthesis method. Lentivirus was produced using a three-plasmid system. Stable overexpression cell lines were generated via lentiviral infection. Briefly, CT26 cells in logarithmic growth phase were seeded at a density of 1×10⁵ cells/mL, with 1 mL per well in a 12-well plate. The viral solution was added to the cell culture plate and incubated in a 37°C incubator with 5% CO₂ for 24 hours. After incubation, the supernatant was discarded, complete medium (DMEM + 10% FBS) was added, and the plate was incubated for another 48 hours. After this incubation, the supernatant was discarded, and the cell pool was subjected to pressure selection using 5 µg/mL puromycin for at least 3 days. Subsequently, the cells were gradient diluted and seeded at a dilution of 1 cell per well in a 96-well cell culture plate. Single clones were expanded and subjected to flow cytometry detection. If the positive rate of a clone was below 90% after detection, the puromycin concentration was increased for further pressure selection to obtain a clone with a positive rate greater than 90%. Finally, after further screening using a higher concentration of puromycin (16 µg/mL), a cell pool with a high positive rate of up to 96.26% was obtained, successfully constructing the stable cell line CT26-hITGB3.

The culture supernatant of the constructed cell line CT26-ITGB3 was tested for mycoplasma using a mycoplasma detection kit (Lonza MycoAlert^{™} mycoplasma detection kit, Cat. No. LT07-318) according to the manufacturer's instructions. The test results showed that the cells were negative for mycoplasma.

### 2.2 Study of the Anti-Tumor Efficacy of Test Substances in Mouse Colon Cancer CT26-hITGB3 Cell Line of BALB/c Mouse Subcutaneous Xenograft Model

The mouse colon cancer cell line CT26-hITGB3 was cultured in an RPMI-1640 medium supplemented with 10% FBS in a 37°C incubator with 5% CO₂. Cells were collected before continuous passage beyond ten generations. Cells (2.5 × 10⁵) were suspended in 100 µL PBS and subcutaneously injected into the right back near the armpit of female BALB/c mice. Mice were anesthetized with 3-4% isoflurane prior to inoculation. When the tumor volume reached an average of approximately 50-60 mm³, mice were randomly divided into 4 groups (n=6 per group) based on tumor volume and body weight. The day of grouping and dosing was defined as Day 0. Grouping and dosing regimens are shown in Table 1.

The body weight and tumor volume of the mouse were measured and recorded twice a week after grouping. Tumor volume (TV) was calculated as follows: TV = (length × width²) / 2. Based on the measured tumor volumes, the relative tumor growth inhibition (TGI) was calculated using the following formula: TGI % = (1 - T/C) × 100%. Where T and C are the tumor volumes (TV) of the experimental group and the vehicle control group, respectively, at a specific time point.

The mean TV value for each group is presented as mean ± standard error of the mean (SEM). Comparisons between groups were performed using Dunnett's multi-comparison test. P-value < 0.05 was considered statistically significant.

### 2.3 Experimental Results

Under these experimental conditions, CT26-hlTGB3 cells exhibited normal growth, and tumor growth proceeded normally. Under these experimental conditions, there was no significant difference in body weight among the experimental groups compared to the vehicle control group at the same time point during the dosing period.

The tumor growth curve in mice is shown in Figure 2. Compared to the vehicle control group at the same time point, on Day 17, the tumor growth inhibition (TGI) for the anti-PD-1 monotherapy group (10 mg/kg) and the HY1272 monotherapy group (10 mg/kg) was 20.2% and 31.7%, respectively. Although a certain anti-tumor trend was observed, there was no significant difference (P < 0.05) compared to the vehicle control group in this experimental system. The TGI for the combination therapy group (10 mg/kg anti-PD-1 + 10 mg/kg HY1272) was 74.8%, showing a significant difference compared to the vehicle control group, the anti-PD-1 monotherapy group, or the HY1272 monotherapy group.

These results indicate that in this colon cancer model, neither anti-PD-1 monotherapy nor HY1272 monotherapy showed significant anti-tumor effects, but their combination exhibited a significant anti-tumor effect. This suggests that HY1272 has a synergistic effect with anti-PD-1 and can significantly enhance the sensitivity of certain colon cancer cells to anti-PD-1 therapy.

It should be understood that although the present disclosure has been described with reference to its preferred embodiments by way of example, it should not be limited to the above examples. For those skilled in the art, various modifications and changes can be made to the present disclosure. The selection and application of specific antibodies can be adjusted and changed according to specific needs. Therefore, for those skilled in the art, several simple substitutions can also be made without departing from the concept and principles of the present disclosure, all of which should be included within the scope of protection of the present disclosure.

## Claims

1. A pharmaceutical combination comprising an anti-PD-1 antibody and an anti-αvβ3 antibody.

2. The pharmaceutical combination according to claim 1, wherein the anti-αvβ3 antibody comprises:
a HCDR1 region having at least 80% identity to the amino acid sequence set forth in SEQ ID NO: 1; a HCDR2 region having at least 80% identity to the amino acid sequence set forth in SEQ ID NO: 2; a HCDR3 region having at least 80% identity to the amino acid sequence set forth in SEQ ID NO: 3; and
a LCDR1 region having at least 80% identity to the amino acid sequence set forth in SEQ ID NO: 4; a LCDR2 region having at least 80% identity to the amino acid sequence set forth in SEQ ID NO: 5; a LCDR3 region having at least 80% identity to the amino acid sequence set forth in SEQ ID NO: 6.

3. The pharmaceutical combination according to claim 2, wherein the anti-αvβ3 antibody comprises:
a HCDR1 region comprising the amino acid sequence set forth in SEQ ID NO: 1; a HCDR2 region comprising the amino acid sequence set forth in SEQ ID NO: 2; a HCDR3 region comprising the amino acid sequence set forth in SEQ ID NO: 3; and
a LCDR1 region comprising the amino acid sequence set forth in SEQ ID NO: 4; a LCDR2 region comprising the amino acid sequence set forth in SEQ ID NO: 5; a LCDR3 region comprising the amino acid sequence set forth in SEQ ID NO: 6;
preferably, the anti-αvβ3 antibody comprises a heavy chain variable region comprising the amino acid sequence set forth in SEQ ID NO: 7 and a light chain variable region comprising the amino acid sequence set forth in SEQ ID NO: 8;
preferably, the anti-αvβ3 antibody is HY1272.

4. The pharmaceutical combination according to any one of claims 1-3, wherein the anti-PD-1 antibody and the anti-αvβ3 antibody are each in a pharmaceutically acceptable form and are administered simultaneously, sequentially, or separately, preferably simultaneously.

5. The pharmaceutical combination according to any one of claims 1-4, wherein the route of administration for the pharmaceutical combination comprises intravenous injection, intraperitoneal injection, intramuscular injection, subcutaneous injection, oral administration, sublingual administration, nasal administration, or transdermal administration, preferably intravenous injection.

6. A use of the pharmaceutical combination according to any one of claims 1-5 in the manufacture of a medicament for treating a patient suffering from a cancer.

7. The use according to claim 6, wherein the cancer is a lung cancer or a colon cancer.

8. The use according to claim 7, wherein the tumor tissue section of the patient is positive for PD-L1 expression, optionally positive for αvβ3 expression.

9. A method for treating a cancer, comprising administering a therapeutically effective amount of the pharmaceutical combination according to any one of claims 1-5 to a patient suffering from a cancer.

10. The method according to claim 9, wherein the cancer is a lung cancer or a colon cancer.

11. The method according to claim 10, wherein the tumor tissue section of the patient is positive for PD-L1 expression, optionally positive for αvβ3 expression.

12. A use of an anti-αvβ3 antibody in the manufacture of a pharmaceutical combination for combined use with an anti-PD-1 antibody.

13. The use according to claim 12, wherein the anti-αvβ3 antibody comprises:
a HCDR1 region having at least 80% identity to the amino acid sequence set forth in SEQ ID NO: 1; a HCDR2 region having at least 80% identity to the amino acid sequence set forth in SEQ ID NO: 2; a HCDR3 region having at least 80% identity to the amino acid sequence set forth in SEQ ID NO: 3; and
a LCDR1 region having at least 80% identity to the amino acid sequence set forth in SEQ ID NO: 4; a LCDR2 region having at least 80% identity to the amino acid sequence set forth in SEQ ID NO: 5; a LCDR3 region having at least 80% identity to the amino acid sequence set forth in SEQ ID NO: 6.

14. The use according to claim 13, wherein the anti-αvβ3 antibody comprises:
a HCDR1 region comprising the amino acid sequence set forth in SEQ ID NO: 1; a HCDR2 region comprising the amino acid sequence set forth in SEQ ID NO: 2; a HCDR3 region comprising the amino acid sequence set forth in SEQ ID NO: 3; and
a LCDR1 region comprising the amino acid sequence set forth in SEQ ID NO: 4; a LCDR2 region comprising the amino acid sequence set forth in SEQ ID NO: 5; a LCDR3 region comprising the amino acid sequence set forth in SEQ ID NO: 6;
preferably, wherein the anti-αvβ3 antibody comprises a heavy chain variable region comprising the amino acid sequence set forth in SEQ ID NO: 7 and a light chain variable region comprising the amino acid sequence set forth in SEQ ID NO: 8;
preferably, wherein the anti-αvβ3 antibody is HY1272.

15. A combination therapy for treating a patient suffering from a cancer, comprising administering a therapeutically effective amount of the anti-αvβ3 antibody according to claim 2 or 3 alone to said patient, as well as administering a therapeutically effective amount of an anti-PD-1 antibody alone.

16. The combination therapy for treating a patient suffering from a cancer according to claim 15, wherein the cancer is a lung cancer or a colon cancer.

17. The combination therapy for treating a patient suffering from a cancer according to claim 16, wherein the tumor tissue section of the patient is positive for PD-L1 expression, optionally positive for αvβ3 expression.

18. The combination therapy according to any one of claims 15-17, wherein the anti-αvβ3 antibody is administered within ±5 minutes of administering the anti-PD-1 antibody.
